(19) Europäisches Patentamt
European Patent Office
Office européen des brevets

(11) Veröffentlichungsnummer: **0 360 163 B1**

# (12) EUROPÄISCHE PATENTSCHRIFT

(45) Veröffentlichungstag der Patentschrift: **12.01.94**

(21) Anmeldenummer: **89117079.7**

(22) Anmeldetag: **15.09.89**

(51) Int. Cl.5: **C07D 239/60**, C07D 403/12, C07D 409/12, A01N 43/54, A01N 43/66, C07D 251/30, C07D 401/12

## (54) Aromatische Carbonsäurederivate und ihre Verwendung zur Bekämpfung unerwünschten Pflanzenwuchses.

(30) Priorität: **22.09.88 DE 3832237**

(43) Veröffentlichungstag der Anmeldung: **28.03.90 Patentblatt 90/13**

(45) Bekanntmachung des Hinweises auf die Patenterteilung: **12.01.94 Patentblatt 94/02**

(84) Benannte Vertragsstaaten: **CH DE ES FR GB IT LI NL**

(56) Entgegenhaltungen:
**EP-A- 0 219 835**
**EP-A- 0 223 406**
**EP-A- 0 249 707**

(73) Patentinhaber: **BASF Aktiengesellschaft**
**Carl-Bosch-Strasse 38**
**D-67063 Ludwigshafen(DE)**

(72) Erfinder: **Rheinheimer, Joachim, Dr.**
**Merziger Strasse 24**
**D-6700 Ludwigshafen(DE)**
Erfinder: **Plath, Peter, Dr.**
**Hans-Balcke-Strasse 13**
**D-6710 Frankenthal(DE)**
Erfinder: **Eicken, Karl, Dr.**
**Am Huettenwingert 12**
**D-6706 Wachenheim(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

**EP 0 360 163 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue aromatische Carbonsäurederivate, Verfahren z ihrer Herstellung und ihre Verwendung zur Bekämpfung unerwünschten Pflanzenwuchses.

Herbizide Benzoesäure- bzw. Pyridincarbonsäurederivate sind bereits in EP-A-223 406, EP-A-249 707 und EP-A-249 708 beschrieben. Acylamino substituierte Pyridincarbonsäureester mit herbizider Wirkung werden in EP-A-219 835 offenbart.

Es wurden nun neue aromatische Carbonsäurederivate der Formel

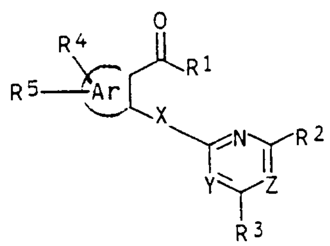

(I)

gefunden, in der der Rest

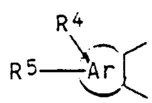

für einen durch $R^4$ und $R^5$ gegebenenfalls substituierten Chinolin-, Naphthalin- oder Benzofuranrest,

$R^1$ für Wasserstoff, Alkylidenaminoxi, welches sich von symmetrischen oder unsymmetrischen, unverzweigten oder verzweigten $C_3$-$C_{20}$-Alkylketonen, oder von $C_8$-$C_{18}$-Alkylphenylketonen ableitet, unsubstituiertes oder durch $C_1$-$C_4$-Alkyl substituiertes $C_4$-$C_{12}$-Cycloalkylidenaminoxi, Succinyliminoxi, unsubstituiertes oder durch $C_1$-$C_3$-Alkyl oder Halogen substituiertes $C_2$-$C_6$-Alkenyloxi oder $C_2$-$C_{10}$-Alkinyloxi, unsubstituiertes oder durch ein Chlor oder Methyl substituiertes Azolyl, $C_1$-$C_4$-Alkylsulfonylamino, $C_3$-$C_{10}$-Alkoxicarbonylalkyloxi, N-Azolyl-$C_1$-$C_4$-alkyloxi, unsubstituiertes oder durch $C_1$-$C_3$-Alkylthio, $C_1$-$C_3$-Alkoxi, Cyano, Phenoxi, $C_1$-$C_3$-Alkylcarbonyl oder Phenylcarbonyl substituiertes $C_1$-$C_{15}$-Alkoxi, unsubstituiertes oder im Phenylteil durch Halogen, Methoxi oder Methyl substituiertes Phenyl-$C_1$-$C_3$-alkoxi, Hydroxyl oder den Rest $O^{\ominus}M^{\oplus}$, wobei $M^{\oplus}$ ein Äquivalent eines Alkalimetall-, Erdalkalimetall- oder organisches Ammoniumions ist.

$R^2$, $R^3$ für $C_1$-$C_5$-Alkyl, $C_1$-$C_5$-Halogenalkyl, $C_1$-$C_5$-Alkoxi, $C_1$-$C_5$-Halogenalkoxi oder $C_1$-$C_4$-Alkylthio,

X für Sauerstoff oder Schwefel,

Y, Z für Stickstoff oder die Methingruppe und

$R^4$, $R^5$ für Wasserstoff, Halogen, $C_1$-$C_6$-Alkyl, $C_1$.$C_6$-Halogenalkyl, Nitro, $C_1$-$C_5$-Alkoxi, unsubstituiertes oder durch $C_1$-$C_3$-Alkyl oder Halogen substituiertes $C_2$-$C_6$-Alkenyloxi, Formyl-, Acetyl-, Pivaloyl- oder Benzoylamino, $C_1$-$C_4$-Alkylamino, $C_1$-$C_4$-Dialkylamino oder Phenylamino stehen.

die ebenso wie ihre landwirtschaftlich anwendbaren Salze herbizid wirksam und gegenüber Kulturpflanzen selektiv sind.

Die Erfindung umfaßt auch Salze der Carbonsäurederivate, die landwirtschaftlich weniger in Frage kommen, jedoch bei der Herstellung der Carbonsäuren der Formel I von Bedeutung sein können.

In Formel I haben die Substituenten $R^1$, $R^2$, $R^3$, $R^4$ und $R^5$ die folgenden Bedeutungen:

$R^1$ steht für Wasserstoff, Alkylidenaminoxi, welches sich von symmetrischen oder unsymmetrischen, unverzweigten oder verzweigten $C_3$-$C_{20}$-Alkylketonen, vorzugsweise von $C_3$-$C_{15}$-Alkylketonen, oder von $C_8$-$C_{18}$-Alkylphenylketonen, vorzugsweise $C_8$-$C_{13}$-Alkylphenylketonen ableitet, gegebenenfalls durch $C_1$-$C_4$-Alkyl, vorzugsweise Methyl, substituiertes $C_4$-$C_{12}$-, vorzugsweise $C_5$-$C_8$-Cycloalkylidenaminoxi, Succinyliminoxi, $C_2$-$C_{10}$-, vorzugsweise $C_2$-$C_6$-Alkenyloxi oder $C_2$-$C_{10}$-, vorzugsweise $C_2$-$C_6$-Alkinyloxi, die durch $C_1$-$C_3$-Alkyl oder Halogen substituiert sein können, gegebenenfalls durch Chlor oder Methyl substituiertes Azolyl, wie Imidazolyl, Dichlorimidazolyl, Pyrazolyl, Dimethylpyrazolyl oder Triazolyl, $C_1$-$C_4$-Alkylsulfonylamino, $C_3$-$C_{10}$-, vorzugsweise $C_3$-$C_7$-Alkoxicarbonylalkoxi, N-Azolyl-$C_1$-$C_4$-alkoxi, gegebenenfalls durch $C_1$-$C_3$-Alkylthio, $C_1$-$C_3$-Alkoxi, Cyano, Phenoxi, $C_1$-$C_3$-Alkylcarbonyl oder Phenylcarbonyl substituiertes $C_1$-$C_{15}$-, vorzugsweise $C_1$-$C_{10}$-Alkoxi, gegebenenfalls im Phenyl-Teil durch Halogen, Methoxi oder Methyl substituiertes Phenyl-$C_1$-$C_3$-alkoxi, Hydroxyl oder den Rest $O^{\ominus}M^{\oplus}$, wobei $M^{\oplus}$ ein Äquivalent eines Alkalimetall-,

Erdalkalimetall- oder eines organischen Ammoniumions ist.

$R^2$ und $R^3$ können gleich oder verschieden sein und $C_1$-$C_5$-Alkyl, $C_1$-$C_5$-Halogenalkyl, beispielsweise $C_1$-$C_3$-Chlor- oder Fluoralkyl, $C_1$-$C_5$-Alkoxi, $C_1$-$C_5$-Halogenalkoxi, beispielsweise $C_1$-$C_2$-Chlor- oder Fluoralkoxi, oder $C_1$-$C_4$-Alkylthio bedeuten.

Unter $R^4$ und $R^5$, die gleich oder verschieden sein können, ist z.B. Wasserstoff, Halogen, Nitro, $C_1$-$C_6$-Alkyl, $C_1$-$C_6$-Halogenalkyl, beispielsweise $C_1$-$C_2$-Chlor- oder Fluoralkyl, $C_1$-$C_5$-Alkoxi, $C_2$-$C_6$-Alkenyloxi, das durch $C_1$-$C_3$-Alkyl oder Halogen substituiert sein kann, $C_1$-$C_4$-Mono- oder Dialkylamino, Formyl-, Acetyl-, Pivaloyl- oder Benzoylamino oder Phenylamino, zu verstehen.

Unter Alkyl oder Alkyl in einer Alkoxigruppe sind je nach der genannten Zahl der Kohlenstoffatome Methyl, Ethyl, Propyl, Isopropyl, Butyl, sek.-Butyl, tert.-Butyl, Pentyl und Isomere, Hexyl und Isomere, Heptyl und Isomere oder Octyl und Isomere zu verstehen. Entsprechend sind auch bei den höheren Homologen die Isomeren jeweils mit einbezogen. Cycloalkyliden bedeutet beispielsweise Cyclobutyliden, Cyclopentyliden, Cyclohexyliden, Cycloheptyliden oder Cyclooctyliden. Alkenyl oder Alkenyl in einer Alkenyloxigruppe kann Vinyl, Propenyl, Butenyl, Pentenyl, Hexenyl oder Heptenyl sein. Aryl bedeutet vorzugsweise Phenyl.

Von den vorstehenden Verbindungen bzw., soweit es sich um Säuren handelt, Salzen, sind bestimmte Gruppen besonders zu erwähnen, nämlich solche, bei denen

$R^1$ für Wasserstoff, gegebenenfalls durch $C_1$-$C_2$-Alkylthio, $C_1$-$C_3$-Alkoxi, Cyano, Phenoxi, $C_1$-$C_3$-Alkylcarbonyl oder Phenylcarbonyl substituiertes $C_1$-$C_{10}$-Alkoxi, gegebenenfalls im Phenyl-Teil durch Halogen, Methoxi oder Methyl substituiertes $C_1$-$C_2$-Phenylalkoxi, Hydroxyl oder den Rest $O^{\ominus}M^{\oplus}$, wobei $M^{\oplus}$ ein Natrium- oder Kaliumion ist, Alkylidenaminoxi, das sich von verzweigten oder unverzweigten $C_3$-$C_{11}$-(Alkylketonen) ableitet, $C_5$-$C_8$-Cycloalkylidenaminoxi, Propargyloxi, verzweigtes oder unverzweigtes $C_2$-$C_6$-Alkenyloxi, $C_2$-$C_6$-Chloralkenyloxi, Methylsulfonylamino, $C_1$-$C_3$-Alkoxicarbonylmethoxi, Imidazolyl, Pyrazolyl oder Triazolyl steht,

$R^2$, $R^3$ gleich oder verschieden sind und für $C_1$-$C_3$-Alkyl, Trifluormethyl, Trichlormethyl, $C_1$-$C_3$-Alkoxi, Difluormethoxi oder $C_1$-$C_3$-Alkylthio stehen,

X für Sauerstoff oder Schwefel,

Y, Z für Stickstoff oder die Methingruppe,

$R^4$ für Wasserstoff, Fluor, Chlor, Brom, $C_1$-$C_4$-Alkyl, Nitro, $C_1$-$C_4$-Alkoxi, Allyloxi, Formylamino, Acetylamino, Pivaloylamino, Benzoylamino, $C_1$-$C_3$-Alkylamino oder Anilino und

$R^5$ für Wasserstoff, Fluor, Chlor, Brom oder Methyl stehen,

und bei denen der Rest

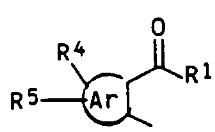

von Naphthalin-1-carbonsäure, Naphthalin-2-carbonsäure, Chinolin-2-carbonsäure, Chinolin-4-carbonsäure, Chinolin-8-carbonsäure oder Derivaten dieser Säuren abgeleitet ist.

Aromatische Carbonsäurederivate der Formel I erhält man in an sich bekannter Weise, indem man ein ortho-Mercapto- oder ortho-Hydrox carbonsäurederivat der Formel II, das entweder bekannt ist oder analog zu bekannten Verbindungen hergestellt werden kann, mit einer heterocyclischen Verbindung der Formel III in Gegenwart einer Base umsetzt.

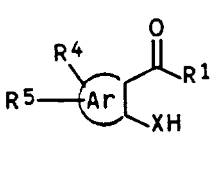

(II)

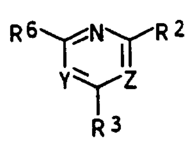

(III)

$R^6$ bedeutet eine Abgangsgruppe, wie Chlor, Brom, Iod, Aryl- oder Alkylsulfonyl, z.B. Toluolsulfonyl oder Methylsulfonyl.

Die Reaktionstemperaturen liegen zwischen -50 und 160 °C, vorzugsweise zwischen 0 und 120 °C. Als Lösungsmittel kommen die üblichen unter den Reaktionsbedingungen inerten Lösungsmittel in Frage.

Die Verbindungen der Formel III mit einem reaktionsfähigen Substituenten $R^6$ sind in den meisten Fällen bekannt oder mit dem allgemeinen Fachwissen leicht herzustellen.

Als Base können 1 bis 10, vorzugsweise 1 bis 3 Äquivalente Alkali- oder Erdalkalimetallhydride wie NaH oder $CaH_2$, Alkalimetallhydroxide wie NaOH oder KOH, Alkalimetallalkoholate wie Kalium-tert.-butylat, Alkalimetallcarbonate wie $Na_2CO_3$ oder $K_2CO_3$, Alkalimetallamide wie $NaNH_2$ oder Lithiumdiisopropylamid oder tertiäre Amine Verwendung finden. Bei Einsatz einer anorganischen Base kann man einen Phasentransferkatalysator zusetzen, um den Umsatz zu erhöhen.

Aromatische Carbonsäurederivate der Formel I, in der $R^1$ Alkylidenaminoxi, unsubstituiertes oder durch Alkyl substituiertes Cycloalkylidenaminoxi, Succinyliminoxi oder unsubstituiertes oder durch Chlor oder Methyl substituiertes Azolyl bedeutet und die übrigen Reste dieselbe Bedeutung haben wie in Formel I gemäß Anspruch 1, lassen sich auch dadurch herstellen, daß man eine Carbonsäure der Formel I in der $R^1$ Hydroxyl ist, zunächst auf übliche Weise in eine aktivierte Form, wie beispielsweise ein Halogenid, gemischtes Anhydrid oder Imidazolid, überführt und dieses dann mit der entsprechenden Hydroxylverbindung umsetzt. Hierbei handelt es sich um Oxime, 1-Hydroxysuccinylimin oder Azole. Im allgemeinen ist es erforderlich, die Reaktion in Gegenwart einer Base durchzuführen. Die beiden Schritte (Aktivierung und Umsetzung) lassen sich beispielsweise dadurch zusammenfassen, daß man die aktivierte Form der Carbonsäure in situ herstellt und direkt weiter umsetzt oder daß man die Carbonsäure in Gegenwart eines wasserabspaltenden Mittels wie beispielsweise eines Carbodiimids auf die Hydroxylverbindung einwirken läßt. Auch dieses zusammengefaßte Verfahren wird von der Erfindung umfaßt.

## Herstellungsbeispiele

### Beispiel 1

Herstellung von 2-(4,6-Dimethoxypyrimidin-2-yl-oxi)-naphthalin-1-aldehyd

8,6 g (0,05 Mol) 2-Hydroxi-naphthalin-1-aldehyd werden in 50 ml getrocknetem Dimethylformamid vorgelegt und bei 0-5°C unter Rühren portionsweise mit 1,5 g (0,05 Mol) 80 %igem Natriumhydrid versetzt. Bei Raumtemperatur gibt man nun 10,9 g (0,05 Mol) 4,6-Dimethoxi-2-methylsulfonylpyrimidin zu, erwärmt auf 90°C und rührt 6 h bei dieser Temperatur nach. Die so erhaltene Lösung wird in Wasser gegossen. Man extrahiert mit Methylenchlorid, wäscht sorgfältig mit Wasser, trocknet über Natriumsulfat und engt unter vermindertem Druck ein. Das Rohprodukt wird durch Ausrühren mit kaltem Toluol weiter gereinigt, man erhält 7,4 g 2-(4,6-Dimethoxypyrimidin-2-yl-oxi)-naphthalin-1-aldehyd vom Schmp. 123-125°C.

### Beispiel 2

Herstellung von 2-(4,6-Dimethoxypyrimidin-2-yl-oxi)-naphthalin-1-carbonsäure

Man löst 4,7 g (0,025 Mol) 2-Hydroxi-naphthalin-1-carbonsäure in 70 ml Methanol und gibt 1,6 g (0,025 Mol) Kaliumyhdroxid (88 %ig) zu. Nach 10 min wird mit 200 ml Toluol versetzt und in der Hitze unter vermindertem Druck eingeengt. Nachdem diese Prozedur einmal wiederholt wurde, löst man das so erhaltene Kaliumsalz in 120 ml getrocknetem Dimethylsulfoxid und gibt bei Raumtemperatur portionsweise 0,75 g (0,025 Mol) 80 %iges Natriumhydrid zu. Es wird 30 min nachgerührt und dann mit 5,5 g (0,025 Mol) 4,6-Dimethoxi-2-methylsulfonylpyrimidin versetzt. Man rührt 2 h bei Raumtemperatur nach und gießt dann in Eiswasser. Es wird mit wenig Essigsäureethylester zweimal extrahiert; der Extrakt wird verworfen. Dann säuert man mit konzentrierter Salzsäure stark an und extrahiert zweimal mit Essigsäureethylester. Der nach dem Trocknen über Natriumsulfat und Einengen verbleibende Rückstand wird mit etwas Diethylether verrührt. Das Produkt wird abfiltriert und unter vermindertem Druck getrocknet. Man erhält 3,7 g 2-(4,6-Dimethoxypyrimidin-2-yl-oxi)-naphthalin-1-carbonsäure vom Schmp. 187-189°C.

### Beispiel 3

Allgemeine Vorschrift zur Herstellung aromatischer Carbonsäuren der Formel I

0,073 Mol einer aromatischen 2-Hydroxi-carbonsäure der Formel I werden in 320 ml getrocknetem Dimethylsulfoxid gelöst und portionsweise mit 16,4 g (0,146 Mol) Kalium-tert.-butylat versetzt, wobei die Temperatur der Reaktionsmischung auf etwa 30°C steigt. Man kühlt auf Raumtemperatur, gibt 0,073 Mol heterocyclische Verbindung der Formel III zu und rührt etwa 1 h bei Raumtemperatur nach. (Die

Beendigung der Reaktion kann mittels Dünnschichtchromatographie überprüft werden.) Die Reaktionsmischung wird in etwa 2 l kaltes Wasser gegossen, mit Salzsäure angesäuert und mit Methyl-tert.-butylether extrahiert. Die organische Phase wird mit etwas Wasser gewaschen, über Natriumsulfat getrocknet und eingeengt. Das zurückbleibende Rohprodukt kann, wenn erforderlich, durch Verrühren mit einem geeigneten Lösungsmittel oder durch Chromatographie an Silica-Gel gereinigt werden.

Beispiel 4

Allgemeine Vorschrift zur Herstellung aromatischer Carbonsäuren der Formel I

5,1 g Kaliumhydroxid und 0,08 Mol einer 2-Hydroxi-carbonsäure der Formel II werden in 80 ml Methanol gelöst, 10 min bei Raumtemperatur gerührt und unter vermindertem Druck eingeengt. Anschließend wird zum Trocknen wiederholt mit Toluol versetzt und dieses bei 50°C unter vermindertem Druck verdampft. Das so erhaltene hellrote Pulver wird in 300 ml Dimethylsulfoxid aufgenommen und bei Raumtemperatur portionsweise mit 2,9 g 80 %igem Natriumhydrid versetzt, wobei eine Gasentwicklung auftritt. Wenn kein Gas mehr frei wird, tropft man eine Lösung von 0,079 Mol heterocyclischer Verbindung der Formel III in 80 ml Dimethylsulfoxid zu und rührt 0,5 h nach. Man gießt in 2 l Wasser, neutralisiert mit Essigsäure und wäscht mit Methylenchlorid. Anschließend wird mit Salzsäure stark angesäuert und mehrmals mit Methyl-tert.-butylether extrahiert. Die organische Phase trocknet man über Natriumsulfat und verdampft das Lösungsmittel unter vermindertem Druck. Die zurückbleibende Substanz kann durch Chromatographie an Silica-Gel gereinigt werden.

Beispiel 5

Allgemeine Vorschrift zur Herstellung aromatischer Carbonsäurederivate der Formel I

3,2 mmol der jeweiligen aromatischen 2-(4,6-Dimethoxypyrimidin-2-yl-oxi)-carbonsäure werden in 20 ml Dimethoxyethan vorgelegt und mit 3,2 mmol Natriumhydrid versetzt, wobei sofort eine Gasentwicklung auftritt. Es wird 1 h bei Raumtemperatur nachgerührt, auf 0°C gekühlt und mit 3,5 mmol Oxalylchlorid versetzt. Man rührt bei 0°C 1 h nach und verdampft dann zur Entfernung des überschüssigen Oxalylchlorids etwa 30 % des Lösungsmittels unter vermindertem Druck. Man gibt nun 4,2 mmol eines Oxims oder einer entsprechenden Hydroxi-verbindung, gelöst in 10 ml Dimethoxiethan, und anschließend 3,2 mmol Pyridin bei 0°C zu und erwärmt innerhalb von 1 h auf Raumtemperatur. Die Mischung wird in 120 ml kaltes Wasser gegossen und mit Methylenchlorid extrahiert. Die organische Phase wird über Natriumsulfat getrocknet und unter vermindertem Druck eingeengt. Die zurückbleibende Substanz kann durch Chromatographie an Silica-Gel weiter gereinigt werden.

Nach diesen Vorschriften können nach Wahl entsprechender Rohstoffe sämtliche Verbindungen der Formel I hergestellt werden; soweit in den nachstehenden Tabellen physikalische Angaben gemacht sind, sind diese Verbindungen der Formel I hergestellt worden. Die anderen aufgeführten Verbindungen sind typische Vertreter der bearbeiteten Substanzklasse.

Die folgenden Verbindungen der Formel I können in analoger Weise erhalten werden:

Tabelle 1

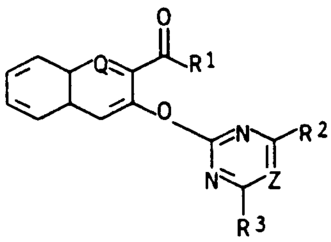

| Nr. | $R^1$ | Q | $R^2$ | $R^3$ | Z | phys. Dat.* |
|---|---|---|---|---|---|---|
| 1.1 | OH | CH | $OCH_3$ | $OCH_3$ | CH | Fp.: 186-187°C |
| 1.2 | $OCH_3$ | CH | $OCH_3$ | $OCH_3$ | CH | δ = 3,75 (3H); 3,80 (6H) 5,75 (1H); 8,55 (1H). |
| 1.3 | $OCH_3$ | CH | $OCH_3$ | $OCH_3$ | N | |
| 1.4 | OH | CH | $OCH_3$ | $OCH_3$ | N | |
| 1.5 | 2-Propyliden-aminoxi | CH | $OCH_3$ | $OCH_3$ | CH | |
| 1.6 | OH | CH | $OCH_3$ | $CF_3$ | CH | |
| 1.7 | OH | N | $OCH_3$ | $OCH_3$ | CH | Fp.: 135°C (Zers.) |
| 1.8 | $OCH_3$ | N | $OCH_3$ | $OCH_3$ | CH | |
| 1.9 | H | N | $OCH_3$ | $OCH_3$ | CH | |
| 1.10 | OH | N | $SCH_3$ | $SCH_3$ | N | |

Tabelle 2

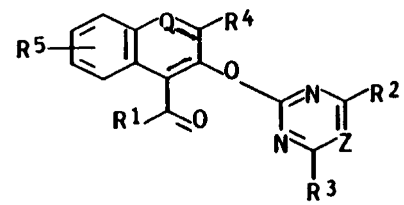

| Nr. | $R^1$ | Q | $R^2$ | $R^3$ | $R^4$ | $R^5$ | Z | phys. Dat.* |
|---|---|---|---|---|---|---|---|---|
| 2.1 | OH | CH | $OCH_3$ | $OCH_3$ | H | H | CH | Fp.: 187-189°C |
| 2.2 | $OCH_3$ | CH | $OCH_3$ | $OCH_3$ | H | H | CH | Fp.: 79- 80°C |
| 2.3 | 1-Imidazolyl | CH | $OCH_3$ | $OCH_3$ | H | H | CH | |
| 2.4 | Propargyloxi | CH | $OCH_3$ | $OCH_3$ | H | H | CH | Fp.: 122-124°C |
| 2.5 | Benzyloxi | CH | $OCH_3$ | $OCH_3$ | H | H | CH | |
| 2.6 | Methylsulfonylamino | CH | $OCH_3$ | $OCH_3$ | H | H | CH | |
| 2.7 | 2-Propylidenaminoxi | CH | $OCH_3$ | $OCH_3$ | H | H | CH | |
| 2.8 | H | CH | $OCH_3$ | $OCH_3$ | H | H | CH | Fp.: 123-125°C |
| 2.9 | OH | N | $OCH_3$ | $OCH_3$ | H | H | CH | Fp.: 189-191°C |
| 2.10 | $OCH_3$ | N | $OCH_3$ | $OCH_3$ | H | H | CH | Fp.: 103-105°C |
| 2.11 | 2-Propylidenaminoxi | N | $OCH_3$ | $OCH_3$ | H | H | CH | |
| 2.12 | $OCH_3$ | N | $OCH_3$ | $OCH_3$ | $CH_3$ | H | CH | Fp.: 142-144°C |
| 2.13 | OH | CH | $OCH_3$ | $OCH_3$ | H | H | N | |
| 2.14 | OH | CH | $SCH_3$ | $SCH_3$ | H | H | N | |
| 2.15 | $OCH_3$ | CH | $OCH_3$ | $OCH_3$ | H | H | N | |
| 2.16 | OH | CH | $CF_3$ | $OCH_3$ | H | H | CH | |
| 2.17 | OH | CH | $OCH_3$ | $OCH_3$ | H | 6-$NO_2$ | CH | |

Tabelle 2 (Fortsetzung)

| Nr. | $R^1$ | Q | $R^2$ | $R^3$ | $R^4$ | $R^5$ | Z | phys. Dat.* |
|---|---|---|---|---|---|---|---|---|
| 2.18 | OH | CH | $OCH_3$ | $OCH_3$ | H | 6-Br | CH | Fp.: 143-145°C |
| 2.19 | $O-CH_2SCH_3$ | CH | $OCH_3$ | $OCH_3$ | H | H | CH | $\delta$ = 2,23 (s); 3,80 (s); 5,36 (s); 5,80 (s) |
| 2.20 | $O-C_2H_4S-C_2H_5$ | CH | $OCH_3$ | $OCH_3$ | H | H | CH | $\delta$ = 1,13 (t); 2,50 (q); 2,68 (t); 3,77 (s) 4,40 (t); 6,05 (s) |
| 2.21 | $O-CH_2-C{\equiv}CH$ | CH | $OCH_3$ | $OCH_3$ | H | H | CH | Fp.: 122-124°C |
| 2.22 | $OC_2H_4-O-CH_3$ | CH | $OCH_3$ | $OCH_3$ | H | H | CH | $\delta$ = 3,20 (3H); 3,50 (m,2H); 3,75 (s,6H); 4,40 (m,2H); 6,05 (s,1H) |

Tabelle 3

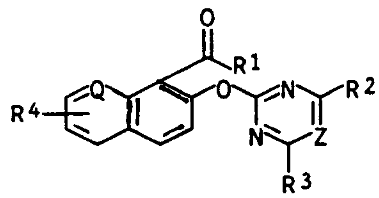

| Nr. | R1 | Q | R2 | R3 | R4 | Z | phys.Dat.* |
|---|---|---|---|---|---|---|---|
| 3.1 | $OCH_3$ | N | $OCH_3$ | $OCH_3$ | $3-CH_3$ | CH | Fp.: 131-132°C |

* Fp.: Schmelzpunkt, $n_D$: Brechungsindex, $\delta$: $^1H$-NMR - chemische Verschiebung in ppm (ausgewählte Signale).

Die aromatischen Carbonsäurederivate der Formel I bzw. die sie enthaltenden herbiziden Mittel sind herbizid wirksam und gegenüber Kulturpflanzen selektiv.

Die Verbindungen der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

Man vermischt 90 Gewichtsteile der Verbindung Nr. 1.1 mit

I.

10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.1 werden in einer Mischung gelöst, die aus 80 Gewichtsteilen Xylol, 10 Gewichtsteilen des Anlagerungsproduktes von 8 bis 10 Mol Ethylenoxid an 1 Mol Öl-säure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 2.8 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 3 Gewichtsteile des Wirkstoffs Nr. 2.9 werden mit 97 Gewichtsteilen feinteiligem Kaolin vermischt. Man erhält auf diese Weise ein Stäubemittel, das 3 Gew.% des Wirkstoffs enthält.

VI. 20 Gewichtsteile des Wirkstoffs Nr. 2.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder vorzugsweise im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5, vorzugsweise 0,01 bis 0,5 kg/ha.

Anwendungsbeispiele

Die herbizide Wirkung der Carbonsäurederivate der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 3,0 kg/ha a.S.

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Amaranthus retroflexus | Zurückgekrümmter Fuchsschwanz |
| Galium aparine | Klettenlabkraut |
| Triticum aestivum | Sommerweizen |

Mit 0,125 bis 3 kg Wirkstoff/ha lassen sich beispielsweise bei Nachauflaufanwendung breitblättrige unerwünschte Pflanzen in Getreide mit den Wirkstoffen Nr. 1.1, 1.2 und 2.1 sehr gut bekämpfen, ohne daß die Kulturpflanzen geschädigt werden.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |

| Botanischer Name | Deutscher Name |
|---|---|
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Carbonsäurederivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäuren, (Het)-Aryloxyphenoxypropionsäuren, deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

**Patentansprüche für folgende Vertragsstaaten : CH, DE, FR, GB, IT, NL**

**1.** Aromatische Carbonsäurederivate der Formel

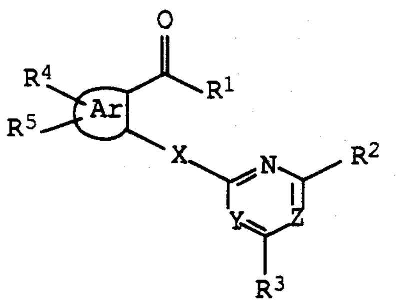

(I)

in der der Rest

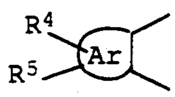

für einen durch $R^4$ und $R^5$ gegebenenfalls substituierten Chinolin-, Naphthalin- oder Benzofuranrest,

$R^1$ für Wasserstoff, Alkylidenaminoxi, welches sich von symmetrischen oder unsymmetrischen, unverzweigten oder verzweigten $C_3$-$C_{20}$-Alkylketonen, oder von $C_8$-$C_{18}$-Alkylphenylketonen ableitet, unsubstituiertes oder durch $C_1$-$C_4$-Alkyl substituiertes $C_4$-$C_{12}$-Cycloalkylidenaminoxi, Succinyliminoxi, unsubstituiertes oder durch $C_1$-$C_3$-Alkyl oder Halogen substituiertes $C_2$-$C_6$-Alkenyloxi oder $C_2$-$C_{10}$-Alkinyloxi, unsubstituiertes oder durch ein Chlor oder Methyl substituiertes Azolyl, $C_1$-$C_4$-Alkylsulfonylamino, $C_3$-$C_{10}$-Alkoxicarbonylalkyloxi, N-Azolyl-$C_1$-$C_4$-alkyloxi, unsubstituiertes oder durch $C_1$-$C_3$-Alkylthio, $C_1$-$C_3$-Alkoxi, Cyano, Phenoxi, $C_1$-$C_3$-Alkylcarbonyl oder Phenylcarbonyl substituiertes $C_1$-$C_{15}$-Alkoxi, unsubstituiertes oder im Phenylteil durch Halogen, Methoxi oder Methyl substituiertes Phenyl-$C_1$-$C_3$-alkoxi, Hydroxyl oder den Rest $O^{\ominus}M^{\oplus}$, wobei $M^{\oplus}$ ein Äquivalent eines Alkalimetall-, Erdalkalimetall- oder organisches Ammoniunmions ist.

$R^2$, $R^3$ für $C_1$-$C_5$-Alkyl, $C_1$-$C_5$-Halogenalkyl, $C_1$-$C_5$-Alkoxi, $C_1$-$C_5$-Halogenalkoxi oder $C_1$-$C_4$-Alkylthio,

X für Sauerstoff oder Schwefel,

Y, Z für Stickstoff oder die Methingruppe und

$R^4$, $R^5$ für Wasserstoff, Halogen, $C_1$-$C_6$-Alkyl, $C_1$.$C_6$-Halogenalkyl, Nitro, $C_1$-$C_5$-Alkoxi, unsubstituiertes oder durch $C_1$-$C_3$-Alkyl oder Halogen substituiertes $C_2$-$C_6$-Alkenyloxi, Formyl-, Acetyl-, Pivaloyl- oder Benzylamino,, $C_1$-$C_4$-Alkylamino, $C_1$-$C_4$-Dialkylamino oder Phenylamino stehen.

**2.** Aromatisches Carbonsäurederivat der Formel I gemäß Anspruch 1, dadurch gekennzeichnet, daß der Rest

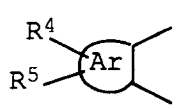

ein durch $R^4$ und $R^5$ gegebenenfalls substituierter Chinolin- oder Naphthalinrest ist.

**3.** Aromatisches Carbonsäurederivat der Formel I gemäß Anspruch 1, dadurch gekennzeichnet, daß
$R^1$ für Wasserstoff, gegebenenfalls durch $C_1$-$C_2$-Alkylthio, $C_1$-$C_3$-Alkoxi, Cyano, Phenoxi, $C_1$-$C_3$-Alkylcarbonyl oder Phenylcarbonyl substituiertes $C_1$-$C_{10}$-Alkoxi, gegebenenfalls im Phenyl-Teil durch Halogen, Methoxi oder Methyl substituiertes $C_1$-$C_2$-Phenylalkoxi, Hydroxyl oder den Rest $O^{\ominus}M^{\oplus}$, wobei $M^{\oplus}$ ein Natrium- oder Kaliumion ist, Alkylidenaminoxi, das sich von verzweigten oder unverzweigten $C_3$-$C_{11}$-(Alkylketonen) ableitet, $C_5$-$C_8$-Cycloalkylidenaminoxi, Propargyloxi, verzweigtes oder unverzweigtes $C_2$-$C_6$-Alkenyloxi, $C_2$-$C_6$-Chloralkenyloxi, Methylsulfonylamino, $C_1$-$C_3$-Alkoxicarbonylmethoxi, Imidazolyl, Pyrazolyl oder Triazolyl steht,
$R^2$, $R^3$ gleich oder verschieden sind und für $C_1$-$C_3$-Alkyl, Trifluormethyl, Trichlormethyl, $C_1$-$C_3$-Alkoxi, Difluormethoxi oder $C_1$-$C_3$-Alkylthio stehen,
X für Sauerstoff oder Schwefel,
Y, Z für Stickstoff oder die Methingruppe,
$R^4$ für Wasserstoff, Fluor, Chlor, Brom, $C_1$-$C_4$-Alkyl, Nitro, $C_1$-$C_4$-Alkoxi, Allyloxi, Formylamino, Acetylamino, Pivaloylamino, Benzoylamino, $C_1$-$C_3$-Alkylamino oder Anilino und
$R^5$ für Wasserstoff, Fluor, Chlor, Brom oder Methyl stehen, und bei denen der Rest

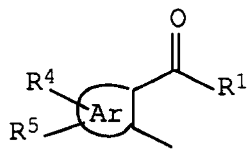

von Naphthalin-1-carbonsäure, Naphthalin-2-carbonsäure, Chinolin-2-carbonsäure, Chinolin-4-carbonsäure, Chinolin-8-carbonsäure, oder Derivaten dieser Säuren abgeleitet ist.

**4.** Verfahren zur Herstellung von aromatischen Carbonsäurederivaten der Formel 1 gemäß Anspruch 1, dadurch gekennzeichnet, daß man ein aromatisches ortho-Mercapto- oder ortho-Hydroxicarbonsäurederivat der Formel II

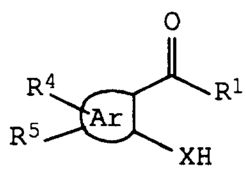

mit einer heterocyclischen Verbindung der Formel III

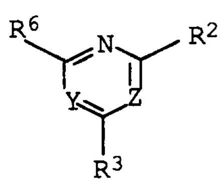

in der $R^6$ eine Abgangsgruppe bedeutet, in an sich üblicher Weise in Gegenwart einer Base umsetzt.

**5.** Verfahren zur Herstellung von aromatischen Carbonsäurederivaten der Formel I gemäß Anspruch 1, in der $R^1$ Alkylidenaminoxi, unsubstituiertes oder durch Alkyl substituiertes Cycloalkylidenaminoxi, Succinyliminoxi oder unsubstituiertes oder durch Chlor oder Methyl substituiertes Azolyl bedeutet, dadurch gekennzeichnet, daß man ein Carbonsäurederivat der Formel I, in der $R^1$ Hydroxyl ist, in das Halogenid oder eine andere aktivierte Form der Carbonsäure überführt und dieses in an sich üblicher Weise gegebenenfalls in Gegenwart einer Base mit einem Oxim, N-Hydroxisuccinylimin oder einem Azol umsetzt.

6. Herbizides Mittel, gekennzeichnet durch einen Gehalt an inerten Trägerstoffen und mindestens einem aromatischen Carbonsäurederivat der Formel I gemäß Anspruch 1.

7. Herbizides Mittel gemäß Anspruch 6, gekennzeichnet durch einen Gehalt an mindestens einem aromatischen Carbonsäurederivat der Formel I gemäß Anspruch 3.

8. Verfahren zur Bekämpfung unerwünschten Pflanzenwuchses, dadurch gekennzeichnet, daß man eine herbizide wirksame Menge einer Verbindung der Formel I gemäß Anspruch 1 auf die Pflanzen oder deren Lebensraum einwirken läßt.

9. Verfahren gemäß Anspruch 8, dadurch gekennzeichnet, daß man eine herbizide wirksame Menge einer Verbindung der Formel I gemäß Anspruch 3 auf die Pflanzen oder deren Lebensraum einwirken läßt.

**Patentansprüche für folgenden Vertragsstaat : ES**

1. Verfahren zur Herstellung von aromatischen Carbonsäurederivaten der Formel

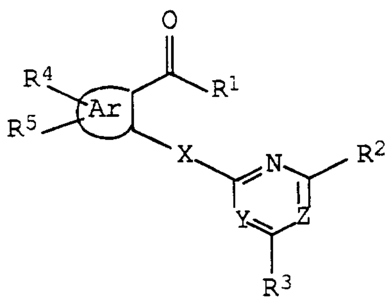

(I)

in der der Rest

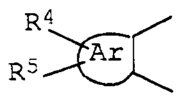

für einen durch $R^4$ und $R^5$ gegebenenfalls substituierten Chinolin-, Naphthalin- oder Benzofuranrest,
$R^1$ für Wasserstoff, Alkylidenaminoxi, welches sich von symmetrischen oder unsymmetrischen, unverzweigten oder verzweigten $C_3$-$C_{20}$-Alkylketonen, oder von $C_8$-$C_{18}$-Alkylphenylketonen ableitet, unsubstituiertes oder durch $C_1$-$C_4$-Alkyl substituiertes $C_4$-$C_{12}$-Cycloalkylidenaminoxi, Succinyliminoxi, unsubstituiertes oder durch $C_1$-$C_3$-Alkyl oder Halogen substituiertes $C_2$-$C_6$-Alkenyloxi oder $C_2$-$C_{10}$-Alkinyloxi, unsubstituiertes oder durch ein Chlor oder Methyl substituiertes Azolyl, $C_1$-$C_4$-Alkylsulfonylamino, $C_3$-$C_{10}$-Alkoxicarbonylalkyloxi, N-Azolyl-$C_1$-$C_4$-alkyloxi, unsubstituiertes oder durch $C_1$-$C_3$-Alkylthio, $C_1$-$C_3$-Alkoxi, Cyano, Phenoxi, $C_1$-$C_3$-Alkylcarbonyl oder Phenylcarbonyl substituiertes $C_1$-$C_{15}$-Alkoxi, unsubstituiertes oder im Phenylteil durch Halogen, Methoxi oder Methyl substituiertes Phenyl-$C_1$-$C_3$-alkoxi, Hydroxyl oder den Rest $O^{\ominus}M^{\oplus}$, wobei $M^{\oplus}$ ein Äquivalent eines Alkalimetall-, Erdalkalimetall- oder organisches Ammoniumions ist.
$R^2$, $R^3$ für $C_1$-$C_5$-Alkyl, $C_1$-$C_5$-Halogenalkyl, $C_1$-$C_5$-Alkoxi, $C_1$-$C_5$-Halogenalkoxi oder $C_1$-$C_4$-Alkylthio,
X für Sauerstoff oder Schwefel,
Y, Z für Stickstoff oder die Methingruppe und
$R^4$, $R^5$ für Wasserstoff, Halogen, $C_1$-$C_6$-Alkyl, $C_1$.$C_6$-Halogenalkyl, Nitro, $C_1$-$C_5$-Alkoxi, unsubstituiertes oder durch $C_1$-$C_3$-Alkyl oder Halogen substituiertes $C_2$-$C_6$-Alkenyloxi, Formyl-, Acetyl-, Pivaloyl- oder Benzoylamino,, $C_1$-$C_4$-Alkylamino, $C_1$-$C_4$-Dialkylamino oder Phenylamino stehen,
dadurch gekennzeichnet, daß man ein aromatisches ortho-Mercapto- oder ortho-Hydroxi-carbonsäurederivat der Formel II

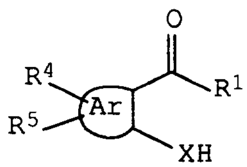

(I)

mit einer heterocyclischen Verbindung der Formel III

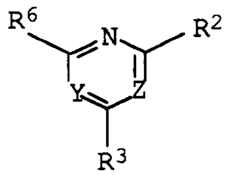

(III),

in der $R^6$ eine Abgangsgruppe bedeutet, in an sich üblicher Weise in Gegenwart einer Base umsetzt.

2. Verfahren zur Herstellung von aromatischen Carbonsäurederivaten der Formel I gemäß Anspruch 1, in der $R^1$ Alkylidenaminoxi, Cycloalkylidenaminoxi, Succinyliminoxi oder Azolyl, jeweils wie in Anspruch 1 definiert, bedeutet, dadurch gekennzeichnet, daß man ein Carbonsäurederivat der Formel I, in der $R^1$ Hydroxyl ist, in das Halogenid oder eine andere aktivierte Form der Carbonsäure überführt und dieses in an sich üblicher Weise gegebenenfalls in Gegenwart einer Base mit einem Oxim, N-Hydroxisuccinylimin oder einem Azol umsetzt.

3. Herbizide Mittel, gekennzeichnet durch eine Gehalt an 0,1 bis 95 Gew.% eines aromatischen Carbonsäurederivats der Formel I gemäß Anspruch 1 und inerten Trägerstoffen.

**Claims**
**Claims for the following Contracting States : CH, DE, FR, GB, IT, LI, NL**

1. An aromatic carboxylic acid derivative of the formula

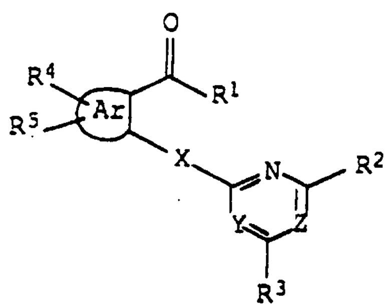

(I)

where the radical

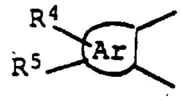

is a quinoline, naphthalene or benzofuran radical which is unsubstituted or substituted by $R^4$ and $R^5$,
$R^1$ is hydrogen, or alkylideneaminoxy which is derived from symmetrical or asymmetrical, straight-chain or branched $C_3$-$C_{20}$-alkyl ketones or from $C_8$-$C_{18}$-alkyl phenyl ketones, or is unsubstituted or $C_1$-$C_4$-alkyl-substituted $C_4$-$C_{12}$-cycloalkylideneaminoxy, succinyliminoxy, unsubstituted or $C_1$-$C_3$-alkyl- or halogen-substituted $C_2$-$C_6$-alkenyloxy or $C_2$-$C_{10}$-alkynyloxy, unsubstituted or chlorine- or methyl-substituted azolyl, $C_1$-$C_4$-alkylsulfonylamino, $C_3$-$C_{10}$-alkoxycarbonylalkyloxy or N-azolyl-$C_1$-$C_4$-alkyloxy, or is $C_1$-

$C_{15}$-alkoxy which is unsubstituted or substituted by $C_1$-$C_3$-alkylthio, $C_1$-$C_3$-alkoxy, cyano, phenoxy, $C_1$-$C_3$-alkylcarbonyl or phenylcarbonyl, or is phenyl-$C_1$-$C_3$-alkoxy which is unsubstituted or substituted in the phenyl moiety by halogen, methoxy or methyl, or is hydroxyl or a radical $O^{\ominus}M^{\oplus}$, where $M^{\oplus}$ is one equivalent of an alkali metal, alkaline earth metal or organic ammonium ion,
$R^2$ and $R^3$ are each $C_1$-$C_5$-alkyl, $C_1$-$C_5$-haloalkyl, $C_1$-$C_5$-alkoxy, $C_1$-$C_5$-haloalkoxy or $C_1$-$C_4$-alkylthio,
X is oxygen or sulfur,
Y and Z are each nitrogen or the methine group and
$R^4$ and $R^5$ are each hydrogen, halogen, $C_1$-$C_6$-alkyl, $C_1$-$C_6$-haloalkyl, nitro, $C_1$-$C_5$-alkoxy, unsubstituted or $C_1$-$C_3$-alkyl- or halogen-substituted $C_2$-$C_6$-alkenyloxy, formylamino, acetylamino, pivaloylamino, benzoylamino, $C_1$-$C_4$-alkylamino, $C_1$-$C_4$-dialkylamino or phenylamino.

**2.** An aromatic carboxylic acid derivative of the formula I as claimed in claim 1, wherein the radical

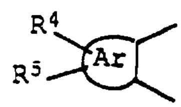

is a quinoline or naphthalene radical which is unsubstituted or substituted by $R^4$ and $R^5$.

**3.** An aromatic carboxylic acid derivative of the formula I as claimed in claim 1, wherein
$R^1$ is hydrogen or $C_1$-$C_{10}$-alkoxy which is unsubstituted or substituted by $C_1$- or $C_2$-alkylthio, $C_1$-$C_3$-alkoxy, cyano, phenoxy, $C_1$-$C_3$-alkylcarbonyl or phenylcarbonyl, or is $C_1$- or $C_2$-phenylalkoxy which is unsubstituted or substituted in the phenyl moiety by halogen, methoxy or methyl, or is hydroxyl or a radical $O^{\ominus}M^{\oplus}$, where $M^{\oplus}$ is a sodium or potassium ion, or $R^1$ is alkylideneaminoxy which is derived from branched or straight-chain $C_3$-$C_{11}$-alkyl ketones, or $R^1$ is $C_5$-$C_8$-cycloalkylideneaminoxy, propargyloxy, branched or straight-chain $C_2$-$C_6$-alkenyloxy, $C_2$-$C_6$-chloroalkenyloxy, methylsulfonylamino, $C_1$-$C_3$-alkoxycarbonylmethoxy, imidazolyl, pyrazolyl or triazolyl,
$R^2$ and $R^3$ are identical or different and are each $C_1$-$C_3$-alkyl, trifluoromethyl, trichloromethyl, $C_1$-$C_3$-alkoxy, difluoromethoxy or $C_1$-$C_3$-alkylthio,
X is oxygen or sulfur,
Y and Z are each nitrogen or the methine group,
$R^4$ is hydrogen, fluorine, chlorine, bromine, $C_1$-$C_4$-alkyl, nitro, $C_1$-$C_4$-alkoxy, allyloxy, formylamino, actylamino, pivaloylamino, benzoylamino, $C_1$-$C_3$-alkylamino or anilino and
$R^5$ is hydrogen, fluorine, chlorine, bromine or methyl, and in which the radical

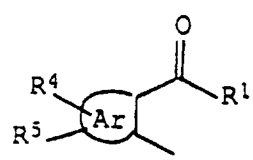

is derived from naphthalene-1-carboxylic acid, naphthalene-2-carboxylic acid, quinoline-2-carboxylic acid, quinoline-4-carboxylic acid, quinoline-8-carboxylic acid or derivatives of these acids.

**4.** A process for the preparation of an aromatic carboxylic acid derivative of the formula I as claimed in claim 1, wherein an aromatic ortho-mercapto- or ortho-hydroxycarboxylic acid derivative of the formula II

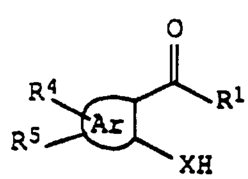

is reacted with a heterocyclic compound of the formula III

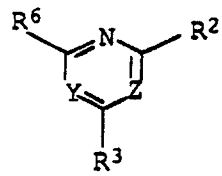

where $R^6$ is a leaving group, in a conventional manner in the presence of a base.

**5.** A process for the preparation of an aromatic carboxylic acid derivative of the formula I as claimed in claim 1, where $R^1$ is alkylideneaminoxy, unsubstituted or alkyl-substituted cycloalkylideneaminoxy, succinyliminoxy or unsubstituted or chlorine- or methyl-subsittuted azolyl, wherein a carboxylic acid derivative of the formula I, where $R^1$ is hydroxyl, is converted into the halide or another activated form of the carboxylic acid and this is reacted in a conventional manner, in the presence or absence of a base, with an oxime, an N-hydroxysuccinylimine or an azole.

**6.** A herbicide which contains inert carriers and at least one aromatic carboxylic acid derivative of the formula I as claimed in claim 1.

**7.** A herbicide as claimed in claim 6, which contains at least one aromatic carboxylic acid derivative of the formula I as claimed in claim 3.

**8.** A method for controlling undesirable plant growth, wherein a herbicidal amount of a compound of the formula I as claimed in claim 1 is allowed to act on the plants or on their habitat.

**9.** A method as claimed in claim 8, wherein a herbicidal amount of a compound of the formula I as claimed in claim 3 is allowed to act on the plants or on their habitat.

**Claims for the following Contracting State : ES**

**1.** A process for the preparation of an aromatic carboxylic acid derivative of the formula

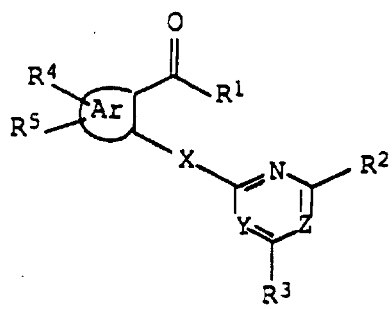

(I)

where the radical

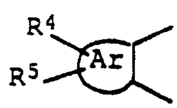

is a quinoline, naphthalene or benzofuran radical which is unsubstituted or substituted by $R^4$ and $R^5$,
$R^1$ is hydrogen, or alkylideneaminoxy which is derived from symmetrical or asymmetrical, straight-chain or branched $C_3$-$C_{20}$-alkyl ketones or from $C_8$-$C_{18}$-alkyl phenyl ketones, or is unsubstituted or $C_1$-$C_4$-alkyl-substituted $C_4$-$C_{12}$-cycloalkylideneaminoxy, succinyliminoxy, unsubstituted or $C_1$-$C_3$-alkyl- or halogen-substituted $C_2$-$C_6$-alkenyloxy or $C_2$-$C_{10}$-alkynyloxy, unsubstituted or chlorine- or methyl-substituted azolyl, $C_1$-$C_4$-alkylsulfonylamino, $C_3$-$C_{10}$-alkoxycarbonylalkyloxy or N-azolyl-$C_1$-$C_4$-alkyloxy, or is $C_1$-$C_{15}$-alkoxy which is unsubstituted or substituted by $C_1$-$C_3$-alkylthio, $C_1$-$C_3$-alkoxy, cyano, phenoxy, $C_1$-

$C_3$-alkylcarbonyl or phenylcarbonyl, or is phenyl-$C_1$-$C_3$-alkoxy which is unsubstituted or substituted in the phenyl moiety by halogen, methoxy or methyl, or is hydroxyl or a radical $O^{\ominus}M^{\oplus}$, where $M^{\oplus}$ is one equivalent of an alkali metal, alkaline earth metal or organic ammonium ion,
$R^2$ and $R^3$ are each $C_1$-$C_5$-alkyl, $C_1$-$C_5$-haloalkyl, $C_1$-$C_5$-alkoxy, $C_1$-$C_5$-haloalkoxy or $C_1$-$C_4$-alkylthio,
X is oxygen or sulfur,
Y and Z are each nitrogen or the methine group and
$R^4$ and $R^5$ are each hydrogen, halogen, $C_1$-$C_6$-alkyl, $C_1$-$C_6$-haloalkyl, nitro, $C_1$-$C_5$-alkoxy, unsubstituted or $C_1$-$C_3$-alkyl- or halogen-substituted $C_2$-$C_6$-alkenyloxy, formylamino, acetylamino, pivaloylamino, benzoylamino, $C_1$-$C_4$-alkylamino, $C_1$-$C_4$-dialkylamino or phenylamino,
wherein an aromatic ortho-mercapto- or ortho-hydroxycarboxylic acid derivative of the formula II

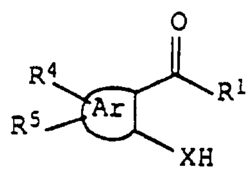

(II)

is reacted with a heterocyclic compound of the formula III

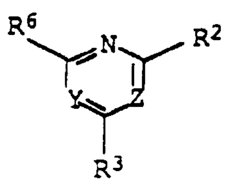

(III),

where $R^6$ is a leaving group, in a conventional manner in the presence of a base.

**2.** A process for the preparation of an aromatic carboxylic acid derivative of the formula I as claimed in claim 1, where $R^1$ is alkylideneaminoxy, cycloalkylideneaminoxy, succinyliminoxy or azolyl, in each case as defined in claim 1, wherein a carboxylic acid derivative of the formula I, where $R^1$ is hydroxyl, is converted into the halide or another activated form of the carboxylic acid and this is reacted in a conventional manner, in the presence or absence of a base, with an oxime, an N-hydroxysuccinylimine or an azole.

**3.** A herbicide which contains from 0.1 to 95% by weight of an aromatic carboxylic acid derivative of the formula I as claimed in claim 1 and inert carriers.

## Revendications
## Revendications pour les Etats contractants suivants : CH, DE, FR, GB, IT, LI, NL

**1.** Dérivés d'acides carboxyliques aromatiques de formule

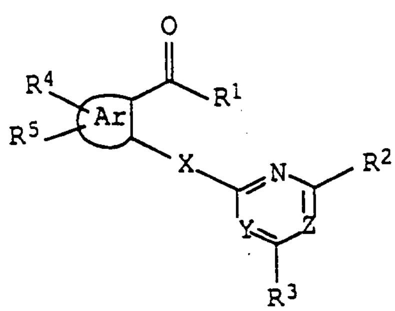

(I)

dans laquelle le reste

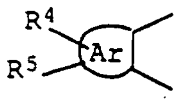

représente un radical de quinoléine, naphtalène ou benzofuranne éventuellement substitué par $R^4$ et $R^5$,

$R^1$ représente l'hydrogène, un groupe alkylidène-aminoxy dérivant d'alkylcétones symétriques ou asymétriques, ramifié ou non ramifiées, en C3-C20, ou d'alkylphénylcétones en C8-C18, un groupe (cycloalkylidène en C4-C12)-aminoxy non substitué ou substitué par un groupe alkyle en C1-C4, un groupe succinyliminoxy, un groupe alcényloxy en C2-C6 ou alcynyloxy en C2-C10, non substitué ou substitué par des groupes alkyle en C1-C3 ou des halogènes, un groupe azolyle non substitué ou substitué par un atome de chlore ou un groupe méthyle, un groupe alkylsulfonyloxy en C1-C4, alcoxycarbonylalkyloxy en C3-C10, N-azolyl-alkyloxy en C1-C4, un groupe alcoxy en C1-C15 non substitué ou substitué par des groupes alkylthio en C1-C3, alcoxy en C1-C3, cyano, phénoxy, alkylcarbonyle en C1-C3 ou phénylcarbonyle, un groupe phényl-alcoxy en C1-C3 non substitué ou substitué dans la partie phényle par des halogènes, des groupes méthoxy ou méthyle, un groupe hydroxy ou le groupe $O^{\ominus}M^{\oplus}$ dans lequel $M^{\oplus}$ représente un équivalent d'un on de métal alcalin, de métal alcalino-terreux ou d'ammonium organique,

$R^2$, $R^3$ représentent des groupes alkyle en C1-C5, halogénoalkyle en C1-C5, alcoxy en C1-C5, halogénoalcoxy en C1-C5 ou alkylthio en C1-C4,

X représente l'oxygène ou le soufre,

Y, Z représentent l'azote ou le groupe méthine, et

$R^4$, $R^5$ représentent l'hydrogène, des halogènes, des groupes alkyle en C1-C6, halogénoalkyle en C1-C6, nitro, alcoxy en C1-C5, alcényloxy en C2-C6 non substitué ou substitué par des groupes alkyle en C1-C3 ou des halogènes, des groupes formyl-, acétyl, pivaloyl- ou benzoyl-amino, alkylamino en C1-C4, dialkylamino en C1-C4 ou phénylamino.

**2.** Dérivé d'acide carboxylique aromatique de formule I de la revendication 1, caractérisé en ce que le reste :

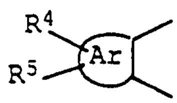

représente un radical de quinoléine ou de naphtalène éventuellement substitué par $R^4$ ou $R^5$.

**3.** Dérivé d'acide carboxylique aromatique de formule I de la revendication 1, caractérisé en ce que :

$R^1$ représente l'hydrogène, un groupe alcoxy en C1-C10 éventuellement substitué par des groupes alkylthio en C1-C2, alcoxy en C1-C3, cyano, phénoxy, alkylcarbonyle en C1-C3 ou phénylcarbonyle, un groupe phénylalcoxy en C1-C2 éventuellement substitué dans la partie phényle par des halogènes, des groupes méthoxy ou méthyle, un groupe hydroxy ou le groupe $O^{\ominus}M^{\oplus}$ dans lequel $M^{\oplus}$ représente un ion sodium ou potassium, un groupe alkylidène-aminoxy dérivant d'alkylcétones ramifiées ou non ramifiées en C3-C11, un groupe cycloalkylidène-aminoxy en C5-C8, propargyloxy, alcényloxy à chaîne droite ou ramifiée en C2-C6, chloralcényloxy en C2-C6, méthylsulfonylamino, (alcoxy en C1-C3)-carbonylméthoxy, imidazolyle, pyrazolyle ou triazolyle,

$R^2$, $R^3$, ayant des significations identiques ou différentes, représentent chacun un groupe alkyle en C1-C3, trifluorométhyle, trichlorométhyle, alcoxy en C1-C3, difluorométhoxy ou alkylthio en C1-C3,

X représente l'oxygène ou le soufre,

Y, Z représentent l'azote ou le groupe méthine,

$R^4$ représente l'hydrogène, le fluor, le chlore, le brome, un groupe alkyle en C1-C4, nitro, alcoxy en C1-C4, allyloxy, formylamino, acétylamino, pivaloylamino, benzoylamino, alkylamino en C1-C3 ou anilino, et

$R^5$ représente l'hydrogène, le fluor, le chlore, le brome ou un groupe méthyle, et le groupe

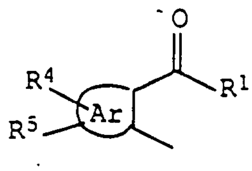

dérive de l'acide naphtalène-1-carboxylique, de l'acide 2-naphtalène-carboxylique, de l'acide quinoléine-2-carboxylique, de l'acide quinoléine-4-carboxylique,de l'acide quinoléine-8-carboxylique ou de dérivés de ces acides.

**4.** Procédé de préparation des dérivés d'acides carboxyliques aromatiques de formule I de la revendication 1, caractérisé en ce que l'on fait réagir de la manière habituelle en présence d'une base un dérivé ortho-mercapto- ou ortho-hydroxy carboxylique aromatique de formule II

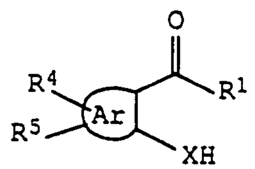

avec un composé hétérocyclique de formule III

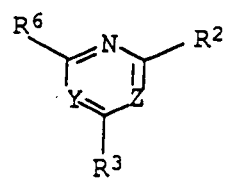

dans laquelle $R^6$ représente un groupe éliminable.

**5.** Procédé de préparation des dérivés d'acides aromatiques carboxyliques de formule I de la revendication 1 dans laquelle $R^1$ représente un groupe alkylidène-aminoxy, cycloalkylidène-aminoxy non substitué ou substitué par des groupes alkyle, succinyliminoxy ou azolyle non substitué ou substitué par le chlore ou des groupes méthyle, caractérisé en ce que l'on convertit un dérivé d'acide carboxylique de formule I, dans laquelle $R^1$ représente un groupe hydroxy, en l'halogénure ou une autre forme activée de l'acide carboxylique, qu'on fait réagir de la manière habituelle, éventuellement en présence d'une base, avec une oxime, la N-hydroxysuccinylimine ou un azole.

**6.** Produit herbicide, caractérisé en ce qu'il contient des véhicules inertes et au moins un dérivé d'acide carboxylique aromatique de formule I de la revendication 1.

**7.** Produit herbicide selon la revendication 6, caractérisé en ce qu'il contient au moins un dérivé d'acide carboxylique aromatique de formule I de la revendication 3.

**8.** Procédé pour combattre les croissances de végétaux indésirables, caractérisé en ce que l'on fait agir sur les végétaux ou leur habitat une quantité herbicide efficace d'un composé de formule I de la revendication 1.

**9.** Procédé selon la revendication 8, caractérisé en ce que l'on fait agir sur les végétaux ou leur habitat une quantité herbicide efficace d'un composé de formule I de la revendication 3.

**Revendications pour l'Etat contractant suivant : ES**

1. Procédé de préparation des dérivés d'acides carboxyliques aromatiques de formule

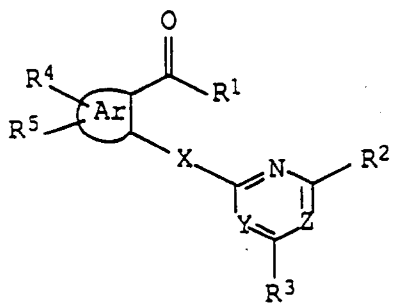

(I)

dans laquelle le reste

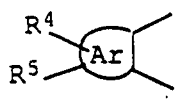

représente un radical de quinoléine, naphtalène ou benzofuranne éventuellement substitué par $R^4$ et $R^5$,
$R^1$ représente l'hydrogène, un groupe alkylidène-aminoxy dérivé d'alkylcétones en C3-C20 symétriques ou asymétriques, droites ou ramifiées, ou d'alkylphénylcétones en C8-C18, un groupe cycloalkylidène-aminoxy en C4-C12 non substitué ou substitué par des groupes alkyle en C1-C4, un groupe succinyliminoxy, un groupe alcényloxy en C2-C6 ou alcynyloxy en C2-C10 non substitué ou substitué par des groupes alkyle en C1-C3 ou des halogènes, un groupe azolyle non substitué ou substitué par un atome de chlore ou un groupe méthyle, un groupe alkylsulfonylamino en C1-C4, alcoxycarbonylalkyloxy en C3-C10, N-azolylalkyloxy en C1-C4, alcoxy en C1-C15 non substitué ou substitué par des groupes alkylthio en C1-C3, alcoxy en C1-C3, cyano, phénoxy, alkylcarbonyle en C1-C3 ou phénylcarbonyle, un groupe phényl-alcoxy en C1-C3 non substitué ou substitué dans la partie phényle par des halogènes, des groupes méthoxy ou méthyle, un groupe hydroxy ou le groupe $O^{\ominus}M^{\oplus}$ dans lequel $M^{\oplus}$ représente un équivalent d'un on de métal alcalin, alcalino-terreux ou d'ammonium organique,
$R^2$, $R^3$ représentent des groupes alkyle en C1-C5, halogénoalkyle en C1-C5, alcoxy en C1-C5, halogénoalcoxy en C1-C5 ou alkylthio en C1-C4,
X représente l'oxygène ou le soufre,
Y, Z représentent l'azote ou le groupe méthine, et
$R^4$, $R^5$ représentent l'hydrogène, des halogènes, des groupes alkyle en C1-C6, halogénoalkyle en C1-C6, nitro, alcoxy en C1-C5, alcényloxy en C2-C6 non substitué ou substitué par des groupes alkyle en C1-C3, un groupe formyl-, acétyl-, pivaloyl- ou benzoyl-amino, un groupe alkylamino en C1-C4, dialkylamino en C1-C4 ou phénylamino,
caractérisé en ce que l'on fait réagir de la manière habituelle en présence d'une base un dérivé d'acide ortho-mercapto- ou ortho-hydroxy-carboxylique aromatique de formule II

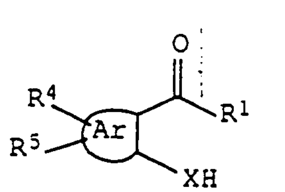

(I)

avec un composé hétérocyclique de formule III

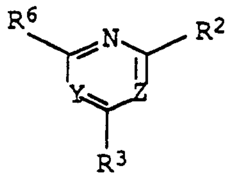

(III),

dans laquelle $R^6$ représente un groupe éliminable.

**2.** Procédé de préparation des dérivés d'acides carboxyliques aromatiques de formule I de la revendication 1 dans laquelle $R^1$ représente un groupe alkylidène-aminoxy, cycloalkylidène-aminoxy, Succinyliminoxy ou azolyle tels que définis chacun dans la revendication 1, caractérisé en ce que l'on convertit un dérivé d'acide carboxylique de formule I, dans laquelle $R^1$ représente un groupe hydroxy en l'halogénure ou en une autre activité de l'acide carboxylique, qu'on fait réagir de la manière habituelle, éventuellement en présence d'une base, avec un oxime, la N-hydroxysuccinylimine ou un azole.

**3.** Produit herbicide, caractérisé en ce qu'il contient de 0,1 % à 95 % en poids d'un dérivé d'acide carboxylique aromatique de formule I de la revendication 1 et des véhicules inertes.